# FASCICULE DE BREVET EUROPEEN

(11) **EP 3 369 452 B1**
(45) Date de publication et mention de la délivrance du brevet: **04.09.2019**
(21) Numéro de dépôt: 18158013.5
(22) Date de dépôt: 22.02.2018
(51) Int. Cl.: A61M 16/00, A61M 16/10, A61M 16/06, A61B 5/00, A61B 5/08, G08C 17/02, G01F 1/32

(54) **APPAREIL DE TRAITEMENT MÉDICAL À DÉBITMÈTRE À OSCILLATION FLUIDIQUE ET MODULE DE COMMUNICATION LONGUE DISTANCE**
MEDIZINISCHES BEHANDLUNGSGERÄT MIT DURCHFLUSSMESSER NACH DEM SCHWINGSTRAHLPRINZIP UND TELEKOMMUNIKATIONSMODUL MIT GROSSER REICHWEITE
MEDICAL TREATMENT APPARATUS WITH FLUIDIC OSCILLATION FLOWMETER AND LONG-DISTANCE COMMUNICATION MODULE

(30) Priorité: 03.03.2017 FR 1751739
(43) Date de publication de la demande: 05.09.2018
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: DUDRET, Stephane, 75015 Paris (FR)
(74) Mandataire: Pittis, Olivier

(56) Documents cités:
- EP-A1- 2 998 637
- WO-A1-93/22627
- WO-A1-2012/098303
- WO-A1-2012/114004
- WO-A2-2012/104513
- FR-A1- 3 006 881
- FR-A1- 3 009 788
- FR-A1- 3 019 623

## Description

La présente invention concerne un appareil de traitement médical, notamment de suivi ou d'observance d'un traitement d'oxygénothérapie comprenant un débitmètre à oscillation fluidique et des moyens de communication à longue distance, et une installation d'oxygénothérapie comprenant un tel appareil de traitement médical.

Dans le cadre d'un traitement par oxygénothérapie d'un patient à domicile, on utilise habituellement un appareil ou dispositif de suivi ou d'observance, venant s'insérer entre la source de gaz, typiquement une source d'oxygène, et le patient, de manière à permettre de suivre les consommations d'oxygène par le patient et s'assurer ainsi que celui-ci observe bien son traitement. Un tel dispositif peut être équipé d'un module de communication permettant de transmettre les données à distance, par exemple à un serveur distant.

Ainsi, WO-A-2009/136101 décrit un dispositif de suivi de l'oxygénothérapie chez un patient soigné à son domicile par administration d'oxygène comprenant un boitier traversé par un conduit, un ou plusieurs capteurs de pression, un microprocesseur, une mémoire, une batterie d'alimentation en courant électrique et une antenne radiofréquence.

EP-A-2670463 propose un dispositif analogue incluant en outre un accéléromètre permettant de suivre les besoins variables en oxygène du patient en fonction de son activité physique, en particulier activité normale ou soutenue, faible activité ou repos, ou sommeil par exemple.

Par ailleurs, EP-A-2506766 enseigne un dispositif permettant de suivre la respiration d'un patient comprenant un capteur de pression différentiel agencé sur un conduit de gaz comprenant en outre une configuration interne de type venturi. Ce dispositif est principalement dédié à la détection des apnées ou hypopnées chez un patient traité sous pression positive continue.

En outre, EP-A-2017586 propose un dispositif permettant de suivre la respiration du patient en ventilation normale ou sous pression positive continue. Il comprend un conduit de gaz équipé d'un élément réducteur de diamètre engendrant une perte de charge et un capteur de pression différentielle permettant de déterminer la pression et le débit du gaz.

Actuellement, dans les appareils de débitmétrie utilisables pour le suivi de patients à leur domicile, la remontée de données mesurées peut se faire à distance, en intégrant dans l'appareil un moyen de télécommunication sans fil à courte distance, par exemple de type Bluetooth ou Wifi.

Typiquement, l'appareil envoie les données, par l'intermédiaire d'un deuxième dispositif comprenant des moyens de communication sans fil dits 'courte portée', à une passerelle dédiée, un ordinateur, une tablette ou encore un téléphone intelligent (i.e. smartphone), lequel va ensuite, après les avoir récupérées, les transmettre à un serveur distant, via un réseau de type GSM, 3G, 4G ou internet, tel que décrit par le document EP-A-2291211 par exemple.

Par ailleurs, FR-A-3009788 enseigne un appareil de traitement médical, notamment de suivi ou d'observance d'un traitement d'oxygénothérapie, à débitmètre avec moyens de pilotage et moyens de télécommunication.

On comprend immédiatement que cette manière de procéder n'est pas idéale pour plusieurs raisons.

Tout d'abord, la transmission des données à un serveur distant nécessite obligatoirement l'utilisation d'un dispositif additionnel, ce qui implique soit une immobilisation d'un deuxième dispositif servant de passerelle chez le patient ou l'utilisateur, ou bien une visite régulière d'un professionnel pour récupérer les données et les transmettre ensuite au serveur. Ceci complexifie l'architecture et les coûts globaux liés à l'utilisation de ces dispositifs.

Ensuite, dans le domaine particulier de la santé, afin de prévenir des rechutes des patients, il est important d'avoir un flux en continu de données provenant des patients, c'est-à-dire tous les jours et toutes les semaines par exemple pour pouvoir agir avant détérioration de la situation sanitaire des patients, c'est-à-dire pour pouvoir détecter leurs éventuelles rechutes aussitôt que possible de manière à pouvoir agir précocement et éviter ou minimiser leur (re)hospitalisation.

En outre, les technologies de communications longue distance usuellement employées, basées sur la connexion à un réseau de téléphonie et de données de type 2G, 3G et 4G, nécessitent l'emploi de modems complexes et coûteux, sont consommatrices en énergie et donc peu compatibles avec des appareils autonomes à longue durée de vie, et sont généralement associés à des coûts d'abonnement au service de communication peu compétitifs.

Au vu de cela, le problème qui se pose est de proposer un appareil de traitement médical, notamment de suivi ou d'observance d'un traitement d'oxygénothérapie, comprenant un dispositif de détermination du débit de gaz, c'est-à-dire un dispositif de type débitmètre, plus simplement appelé « débitmètre », et permettant d'assurer une communication à longue distance des données mesurées, lequel appareil soit bien adapté à une utilisation en oxygénothérapie de patients, en particulier à leur domicile, et ne présente pas tout ou partie des problèmes et inconvénients susmentionnés.

La solution de l'invention est alors un appareil de traitement médical, notamment de suivi ou d'observance d'un traitement d'oxygénothérapie comprenant :
- un conduit de gaz apte à acheminer un flux gazeux, typiquement un gaz respiratoire contenant de l'oxygène,
- un débitmètre agencé sur le conduit de gaz,
- au moins un module de pilotage et de traitement de signal, relié électriquement au débitmètre à oscillation fluidique, configuré pour récupérer et traiter les signaux de mesure délivrés par le débitmètre à oscillation fluidique, et
- au moins un module de télécommunication relié électriquement au module de pilotage et de traitement de signal et configuré pour transmettre des signaux de mesure provenant du module de pilotage et de traitement de signal à au moins un réseau de communication, ledit au moins un module de télécommunication comprenant au moins un modem radio associé à au moins une antenne émettrice/réceptrice,
caractérisé en ce que :
- le débitmètre est un débitmètre à oscillation fluidique, et
- ledit au moins un module de télécommunication est configuré pour présenter une sensibilité inférieure ou égale à -110 dBm et une valeur d'affaiblissement de couplage maximal (MCL; pour *Maximum Coupling Loss*) au moins dans le sens montant et éventuellement dans le sens descendant supérieure ou égale à 130 dB avec ledit au moins un réseau de communication considéré.

Dans le cadre de la présente invention, on appelle :
- « sensibilité » (ou « *sensitivity* » en anglais): le niveau minimal de signal en entrée du récepteur permettant de décoder une information avec un taux d'erreur défini par le standard ou le protocole radio implément. Une définition de « sensitivity » est par exemple donnée par le document 802.11 Wireless LAN Fundamentals, Pejman Roshan, Jonathan Leary, Cisco Press, 2003*;* et
- Affaiblissement de Couplage Maximal ou « MCL » (ou *Maximum Coupling Loss* en anglais) : l'atténuation maximale du signal permettant la transmission radio, entre les ports d'antenne respectivement de l'émetteur et du récepteur. Une définition est par exemple donnée dans le document : 3rd Generation Partnership Project; Technical Specification Group Radio Access Network; Evolved Universal Terrestrial Radio Access (E-UTRA); LTE coverage enhancements (Release 11) - 3GPP TR 36.824 F11.0.0; 2012-06*.*

En effet, dans le cadre de la présente invention, le fait que ledit au moins un module de télécommunication soit configuré, à la fois matériellement et en termes de traitements analogiques et numériques mis en oeuvre, pour présenter une sensibilité inférieure ou égale à -110 dBm et une valeur de MCL supérieure ou égale à 130 dB avec les stations de base du réseau de communication considéré permet de réaliser des transmissions de données sur de longues distances, typiquement de 100 m à 30 km environ, selon l'environnement de propagation des signaux considérés, i.e. milieu urbain ou dégagé .

Selon le cas, l'appareil médical de l'invention peut comprendre l'une ou plusieurs des caractéristiques techniques suivantes :
- la sensibilité est inférieure ou égale à -120 dBm.
- la sensibilité est inférieure ou égale à -125 dBm.
- la sensibilité est inférieure ou égale à -130 dBm.
- la valeur de MCL est supérieure ou égale à 135 dB.
- la valeur de MCL est supérieure ou égale à 140 dB.
- la valeur de MCL est supérieure ou égale à 150 dB.
- avantageusement, la sensibilité est inférieure ou égale à -130 dBm et la valeur de MCL est supérieure ou égale à 150 dB.
- ledit au moins modem radio est configuré pour opérer des modulations dans la bande ISM à 433 MHz, à 868 MHz, à 784 MHz, à 915 MHz ou à 2.4 GHz.
- il comprend en outre un premier capteur de pression absolue agencé pour mesurer la pression ambiante et/ou un second capteur de pression absolue agencé pour mesurer la pression absolue dans le conduit.
- il comprend par ailleurs un capteur de température placé en contact fluidique avec le gaz, et placé en amont de la chambre de stabilisation ou, de préférence, en aval de la chambre d'oscillation, de manière à déterminer la température du gaz traversant le débitmètre, et, optionnellement, connaissant la nature chimique du gaz, et une valeur de la pression du gaz mesurée ou estimée à priori, en déduire la densité du gaz grâce à un modèle, telle que la loi des gaz réels.
- ledit au moins un module de pilotage et de traitement de signal est relié électriquement aux premier et/ou second capteurs et configuré pour récupérer et traiter les signaux de mesure délivrés par les premier et second capteurs.
- ledit au moins un module de pilotage et de traitement de signal est relié électriquement au capteur de température et configuré pour récupérer et traiter les signaux de mesure délivrés par le capteur de température.
- le premier capteur de pression absolue et le second capteur de pression absolue sont conçus pour transmettre au module de pilotage et de traitement de signal, des signaux de pression correspondant à des valeurs de pression mesurées.
- le modem radio et l'antenne émettrice/réceptrice permettent de transmettre à distance, c'est-à-dire à longue portée, des signaux provenant du module de pilotage et de traitement de signal, par exemple des signaux traités, un message d'alerte et/ou d'autodiagnostic.
- le modem radio est configuré pour opérer au moins une modulation à étalement de spectre, de préférence au moins une modulation de type LoRa, éventuellement associée à un protocole d'accès de type LoRaWAN.
- le modem radio est configuré pour opérer au moins une modulation à bande ultra-étroite, de préférence au moins une modulation de type Sigfox, éventuellement associée à un protocole d'accès Sigfox.
- le modem radio est configuré pour opérer au moins une modulation et un protocole ou une pile de protocoles associés, sur au moins un réseau choisi parmi les réseaux EC-GSM, NB-IoT et LTE-M
- le modem radio est configuré pour opérer selon un débit de transmission compris entre 100 bits par seconde, comme dans le cas d'un réseau Sigfox, et 1 Mégabit par seconde, comme dans le cas d'un réseau LTE-M Cat0, c'est-à-dire dans tout ou partie de cette plage de débit de transmission.
- le module de télécommunication est configuré pour transmettre des données à une distance de 100 m à 30 km. En effet, dans le cadre de l'invention, la notion de 'longue distance' s'entend comme une portée de 100 m à 1km en milieu urbain dense avec au moins le récepteur ou l'émetteur situé à l'intérieur d'un bâtiment, et/ou une portée de 100 m à 5 km ou plus, voire jusqu'à 30 km, en milieu rural ou en espace dégagé.
- le module de pilotage et de traitement de signal comprend une ou plusieurs cartes électroniques, de préférence incluant un ou plusieurs microprocesseurs, en particulier un ou des microcontrôleurs.
- il comprend en outre un dispositif ou des moyens de mémorisation de données, telle une mémoire flash ou analogue.
- les moyens de mémorisation de données sont incorporés dans le module de pilotage et de traitement de signal.
- le module de télécommunication comprend plusieurs modems radio agencés en parallèles l'un de l'autre, et associés à au moins une antenne émettrice/réceptrice.
- le premier modem radio est dédié à une première technologie radio et le deuxième modem radio est dédié à une seconde technologie radio.
- le module de pilotage et de traitement de signal commande alternativement le premier modem radio et le deuxième modem radio pour transmettre ou recevoir des données par le biais de l'antenne selon la première technologie radio ou, alternativement, selon la seconde technologie radio.
- un commutateur ou « *switch* » permet de commuter l'antenne sur l'un ou l'autre des modems radio.
- le commutateur est commandé par le module de pilotage et de traitement de signal, en particulier par microcontrôleur intégré au module de pilotage et de traitement de signal
- alternativement, le module de télécommunication comprend plusieurs antennes émettrices/réceptrices, de préférence une première et une deuxième antenne, chaque antenne.
- chaque antenne est reliée électriquement à l'un des modems radio, c'est-à-dire que chaque antenne est dédiée à l'un des modems qui lui est propre.
- il comprend un commutateur additionnel configuré pour fournir au module de pilotage et de traitement de signal, une consigne quant à la logique de transmission à utiliser, la technologie à utiliser systématiquement ou la valeur d'un paramètre d'une logique de transmission de manière à permettre une sélection d'un des modems radio, c'est-à-dire le modem radio devant être utilisé pour mettre en oeuvre la consigne.
- il comprend en outre une antenne additionnelle associée à un troisième modem radio relié électriquement au module de pilotage et de traitement de signal, de manière à permettre une réception par le module de pilotage et de traitement de signal, en particulier un microcontrôleur, d'une consigne quant à la logique de transmission à utiliser, la technologie à utiliser systématiquement, ou la valeur d'un paramètre d'une logique de transmission, au moyen d'une technologie de communication à courte portée, en particulier une technologie de communication à courte portée de type WiFi, Bluetooth, Bluetooth Smart/Bluetooth Low Energy, NFC, Ant/Ant+ ou analogue.
- il comprend en outre une source d'énergie, telle une batterie électrique ou une pile, pour alimenter en courant électrique au moins une partie du module de pilotage et de traitement de signal et/ou du module de télécommunication.
- le débitmètre à oscillation fluidique comprend une chambre de stabilisation comprenant un élément stabilisateur de flux et une chambre d'oscillation comprenant un élément à reflux configuré pour créer au moins un tourbillon gazeux oscillant dans la chambre d'oscillation.
- la chambre de stabilisation et la chambre d'oscillation est en communication fluidique avec le conduit de gaz.
- la chambre d'oscillation comprend deux orifices de mesure.
- l'élément stabilisateur de flux a une section de forme générale triangulaire ou quasi triangulaire.
- l'élément à reflux comprend une partie de section semi-circulaire, i.e. une section hémicirculaire, agencée face au conduit de liaison, formant un demi-cylindre s'étend entre le sol et le plafond de la chambre d'oscillation.
- selon un mode de réalisation, un ou plusieurs microphones sont raccordés aux deux orifices de mesure de la chambre d'oscillation de manière à permettre de mesurer indirectement la pression dans la chambre d'oscillation. En effet, le (les) microphone(s) délivre(nt) une tension électrique proportionnelle à la pression subie, c'est-à-dire un (ou des) signal de tension. Ledit au moins un signal de tension est transmis au module de pilotage et de traitement de signal.
- selon un mode de réalisation alternatif, il comprend en outre un ou plusieurs capteurs de pression raccordés aux deux orifices de mesure de la chambre d'oscillation de manière à permettre de mesurer la pression dans la chambre d'oscillation. Le au moins un signal de tension en résultant est transmis au module de pilotage et de traitement de signal.
- selon un autre mode de réalisation alternatif, il comprend en outre un capteur de différence de pression raccordé aux deux orifices de mesure de la chambre d'oscillation de manière à permettre de mesurer la différence de pression entre les points de mesure dans la chambre d'oscillation. Au moins un signal de différence de tension en résultant est alors transmis au module de pilotage et de traitement de signal.
- les deux orifices de mesure de la chambre d'oscillation sont fermés par une membrane fluidiquement étanche.
- l'élément stabilisateur de flux est espacé de la paroi périphérique de la chambre de stabilisation de sorte de créer des passages pour le gaz autour de l'élément stabilisateur de flux. Le flux de gaz traverse alors la chambre de stabilisation en contournant l'élément stabilisateur de flux, c'est-à-dire en passant de part et d'autre de l'élément stabilisateur de flux.
- le gaz est de l'air, de l'oxygène ou un mélange air/oxygène.
- la chambre d'oscillation comprend une paroi périphérique reliant les deux parois parallèles, c'est-à-dire les deux parois agencées en vis-à-vis ou face à face dont l'une porte les deux orifices de mesure. En fait, le gaz traversant le débitmètre est séparé des capteurs par une membrane suffisamment fine qui transmet les variations de pression mais empêche le contact direct du gaz avec les capteurs.
- les deux parois parallèles délimitant la chambre d'oscillation forment le plafond et le sol de la chambre d'oscillation, c'est-à-dire que les deux orifices de mesure sont agencés dans le sol ou le plafond.
- l'élément stabilisateur de flux est configuré pour permettre de rendre le profil de vitesse du gaz en sortie de cet élément en deux dimensions (2D), donc invariable dans la direction perpendiculaire au plan du débitmètre, et de plus symétrique par rapport au plan de symétrie du débitmètre. En effet, le profil de vitesse du gaz arrivant en entrée de cet élément est souvent en 3 dimensions (3D) et dissymétrique. Le fait de changer brutalement la direction de l'écoulement en entrée de cet élément dans une section rectangulaire en plus qui se rétrécit au fur et à mesure qu'on s'approche du conduit de liaison, qui est lui-même de section rectangulaire, permet de transformer l'écoulement 3D en écoulement 2D. D'autre part, la géométrie symétrique par rapport au plan de symétrie de cet élément permet de symétriser aussi le profil de vitesse.
- le conduit de liaison convoie le gaz de la chambre de stabilisation à la chambre d'oscillation en accélérant la vitesse du gaz car la section rectangulaire de passage du gaz est inférieure à celle du passage agencé dans l'élément stabilisateur. En effet, il faut une vitesse de gaz supérieure à une valeur minimale en entrée de la chambre d'oscillateur pour déclencher les oscillations car, en l'absence de vitesse minimale, mesurer le débit de gaz n'est pas possible.
- les deux orifices de mesure sont fermés, c'est-à-dire recouverts, par une membrane fluidiquement étanche. Cette membrane transmet les variations de pression du côté de la chambre d'oscillation vers l'endroit où se trouvent les capteurs, c'est-à-dire microphones ou capteurs de pression ou de différence de pression.
- l'élément à reflux comprend une partie de section semi-cylindrique agencée face au conduit de liaison.
- le gaz pénètre dans la chambre de stabilisation par le premier orifice d'entrée et ressort de la chambre de stabilisation par le premier orifice de sortie.
- le gaz entre dans la chambre d'oscillation par le second orifice d'entrée et ressort de la chambre d'oscillation par le second orifice de sortie.
- le conduit de liaison relie fluidiquement le premier orifice de sortie de la chambre de stabilisation au second orifice d'entrée de la chambre d'oscillation.
- il comprend en outre un ou plusieurs microphones raccordés auxdits deux orifices de mesure de manière à permettre de mesurer la pression dans la chambre d'oscillation, de préférence des microphones.
- chaque orifice de mesure est relié à un microphone.
- un canal d'entrée est relié fluidiquement au premier orifice d'entrée de la chambre de stabilisation. Le canal d'entrée alimente la chambre de stabilisation en gaz.
- un conduit d'évacuation de gaz est en communication fluidique avec le second orifice de sortie de gaz de la chambre d'oscillation de manière à récupérer le gaz sortant de la chambre d'oscillation.
- le canal d'entrée et le conduit d'évacuation de gaz sont en communication fluidique avec le conduit de gaz de l'appareil, sur lequel est agencé le débitmètre.

L'invention concerne aussi une installation d'oxygénothérapie comprenant :
- une source de gaz respiratoire, par exemple un appareil de distribution de gaz ou une bouteille de gaz,
- une interface de distribution de gaz permettant de distribuer le gaz respiratoire à un patient, tel que des canules nasales ou un masque respiratoire, et
- un appareil de traitement médical, en particulier un appareil ou dispositif de suivi ou d'observance d'un traitement d'oxygénothérapie selon la présente invention, tel que décrit dans la présente description.

L'invention va maintenant être mieux comprise grâce à la description détaillée suivante, faite à titre illustratif mais non limitatif, en référence aux Figures annexées parmi lesquelles :
- la Figure 1 est un schéma du principe de fonctionnement d'un débitmètre à oscillation fluidique intégré à un appareil médical selon l'invention,
- la Figure 2 est une représentation tridimensionnelle d'un débitmètre à oscillation fluidique, analogue à celui de la Figure 1,
- la Figure 3 représente une installation d'oxygénothérapie incluant un appareil de suivi ou d'observance selon l'invention,
- la Figure 4 est un schéma de principe de l'architecture générale d'un appareil de traitement médical, à savoir ici un appareil de suivi ou d'observance d'un traitement d'oxygénothérapie, selon un premier mode de réalisation de l'invention,
- la Figure 5 illustre un second mode de réalisation de l'appareil de traitement médical de la Figure 4,
- la Figure 6 illustre un troisième mode de réalisation analogue à celui de la Figure 5,
- la Figure 7 illustre un quatrième mode de réalisation qui est aussi analogue à celui de la Figure 5, et
- la Figure 8 illustre un cinquième mode de réalisation intégrant tout ou partie des éléments des premier (Figure 4) et quatrième (Figure 7) modes de réalisation.

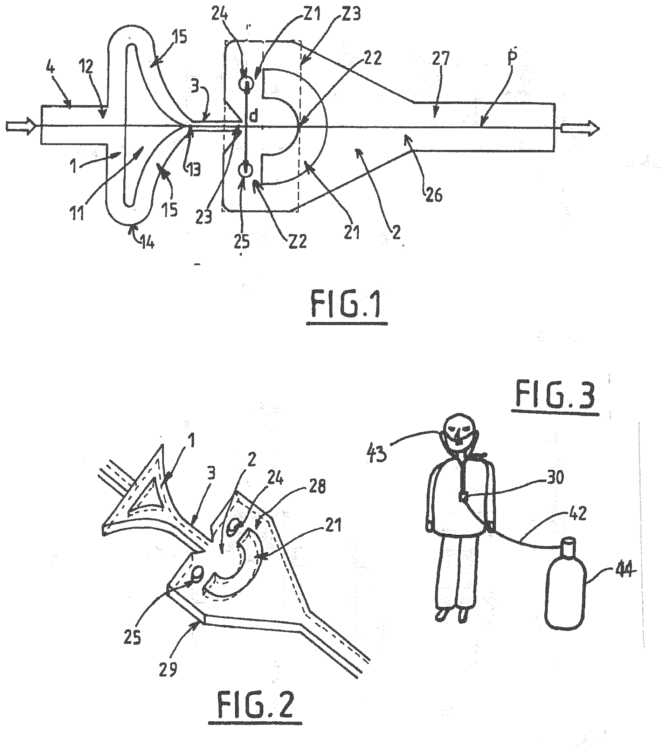

La Figure 1 est un schéma du principe de fonctionnement d'un débitmètre à oscillation fluidique (vue de dessus) pour un appareil de traitement médical selon l'invention, en particulier un appareil d'oxygénothérapie.

Il comprend une chambre de stabilisation 1 dans laquelle est agencé un élément stabilisateur de flux 11, ayant ici une section générale sensiblement triangulaire ou quasi-triangulaire et une face avant, et une chambre d'oscillation 2 comprenant un élément à reflux 21 ayant ici une forme de demi-cylindre (i.e. une section hémicirculaire), lequel est configuré en arc de cercle 22 pour créer un tourbillon ou vortex gazeux oscillant. Le tourbillon oscille en fait entre deux zones Z1, Z2 situées schématiquement au niveau des extrémités du demi-cylindre de l'élément à reflux 21.

L'élément à reflux 21 est pris en sandwich entre deux parois parallèles 28, 29 délimitant la chambre d'oscillation 2 en haut et en bas respectivement (Figure 2), c'est-à-dire formant le plafond et le sol de la chambre d'oscillation 2.

Un conduit de liaison 3 relie fluidiquement la chambre de stabilisation 1 à la chambre d'oscillation 2 de sorte que le gaz qui entre dans la chambre de stabilisation 1, la traverse et alimente ensuite la chambre d'oscillation 2. Le conduit de liaison 3 y débouche en vis-à-vis, c'est-à-dire en face ou en regard, de l'élément à reflux 21comprenant une cavité évidée de section semi-circulaire, i.e. préférentiellement hémicirculaire, ce qui engendre une oscillation du flux et une formation de tourbillons dans les deux zones Z1 et Z2 suscitées. Par exemple, l'élément à reflux 21 peut avoir une forme générale semi-cylindrique comme sur la Figure 1, ou une forme générale substantiellement ou quasi-semi-cylindrique.

Un plan de symétrie P sépare l'ensemble du système, en particulier le conduit de liaison 3, la chambre de stabilisation 1, l'élément stabilisateur de flux 11, la chambre d'oscillation fluidique 2 et l'élément à reflux 21, en deux parties égales et symétriques par rapport à ce plan de symétrie P.

La face avant de l'élément stabilisateur de flux 11 compris dans la chambre de stabilisation 1 est plate et perpendiculaire au plan de symétrie P, donc perpendiculaire à l'axe du conduit de liaison 3.

Une telle configuration est connue et décrite dans la publication : *Yves Le Guer ; Jet confiné, dispersions fluide-particules et mélange chaotique ; Engineering Sciences ; Université de Pau et des Pays de l'Adour; 2005,* et dans le document WO-A-93/22627.

Pour assurer une mesure efficace de la variation de la pression du gaz, en fonction du temps, au sein de la chambre à reflux 2 dans laquelle oscille le flux gazeux en formant des tourbillons gazeux dans les zones Z1, Z2, il convient de positionner les sites de mesure, c'est-à-dire les orifices de mesure 24, 25, reliés à des microphones ou à des capteurs de pression, de préférence des microphones (non représentés), dans le plafond (ou dans le sol) de la chambre à reflux 2, c'est-à-dire approximativement au-dessus des zones Z1, Z2 où se forment les tourbillons, et surtout symétriquement par rapport au plan de symétrie P du débitmètre en respectant impérativement entre eux, une distance d (mesurée entre les axes ou centres des orifices de mesures comprise entre environ 0,5 et 15 mm (cf. Figure 1), de préférence entre environ 0,5 et 10 mm, par exemple de l'ordre d'environ 1 à 6 mm.

Les deux orifices de mesure 24, 25 reliés de préférence à des microphones se situent de façon préférentielle sur un axe perpendiculaire au plan de symétrie P, et de façon préférentielle dans la zone Z3 représentée en pointillés sur la Figure 1. Le positionnement des deux orifices de mesure 24, 25, l'un par rapport à l'autre, ainsi que par rapport à d'autres éléments de la géométrie du système de débitmètre joue un rôle important dans la perception de la fréquence d'oscillation de la pression du tourbillon et par conséquent influence la précision de calcul du débit à partir des valeurs de pression mesurées par les capteurs reliés aux deux orifices de mesure 24, 25.

Les deux orifices de mesure 24, 25 sont préférentiellement fermés par une membrane fluidiquement étanche de manière à assurer le bon fonctionnement des microphones. En fait, la pression dans la chambre d'oscillation 2 se transmet aux microphones, via les deux orifices 24, 25, et au travers des membranes qui recouvrent ces deux orifices 24, 25. De préférence, la membrane a une épaisseur très fine au niveau des capteurs 24 et 25, typiquement de l'ordre de 50 à 500 µm environ ; ailleurs, son épaisseur peut être comprise entre 1 et 2 mm, voire plus.
En fait, en fonctionnement, le flux de gaz circule dans le sens des flèches (=>) représentées en Figure 1.

Le flux de gaz, par exemple de l'oxygène ou de l'air enrichi en oxygène, arrive par un canal d'entrée 4 est relié fluidiquement au premier orifice d'entrée 12 de la chambre de stabilisation 1 et pénètre dans ladite chambre de stabilisation 1, via ce premier orifice d'entrée 12.

Au sein de la chambre de stabilisation 1, le flux est soumis à une stabilisation par l'élément stabilisateur de flux 11, qui est de section se rapprochant de celle triangulaire avec sa base, c'est-à-dire sa face avant la, orientée en vis-à-vis du débouché du canal d'entrée 4, donc en face du premier orifice d'entrée 12. En fait, la section de l'élément stabilisateur de flux 11 est légèrement concave en se rapprochant de plus en plus de l'entrée 13 du conduit 3.

Le flux gazeux contourne donc l'élément stabilisateur de flux 11 en passant dans des passages 15 aménagés de part et d'autre de celui-ci. Les passages 15 sont en fait délimités par la surface externe de l'élément stabilisateur de flux 11 et par la paroi périphérique interne 14 de la chambre de stabilisation 1. En d'autres termes, l'élément stabilisateur de flux 11 est espacé de la paroi périphérique 14 de la chambre de stabilisation 1 de manière à créer des passages 15 pour le gaz autour dudit élément stabilisateur de flux 11.

Le flux gazeux ressort ensuite de la chambre de stabilisation 1 par le premier orifice de sortie 13 et est acheminé par le conduit de liaison 3 qui relie fluidiquement le premier orifice de sortie 13 de la chambre de stabilisation 1 au second orifice d'entrée 23 de la chambre d'oscillation 2.

Les premier et second orifices d'entrée 12, 23 et les premier et second orifices de sortie 13, 26 sont agencés de façon symétrique par rapport au plan de symétrie P.

Le gaz continue ensuite sa course dans la chambre d'oscillation 2 avant d'en ressortir par un conduit d'évacuation de gaz 27 qui est en communication fluidique avec le second orifice de sortie 26 de gaz de la chambre d'oscillation 2.

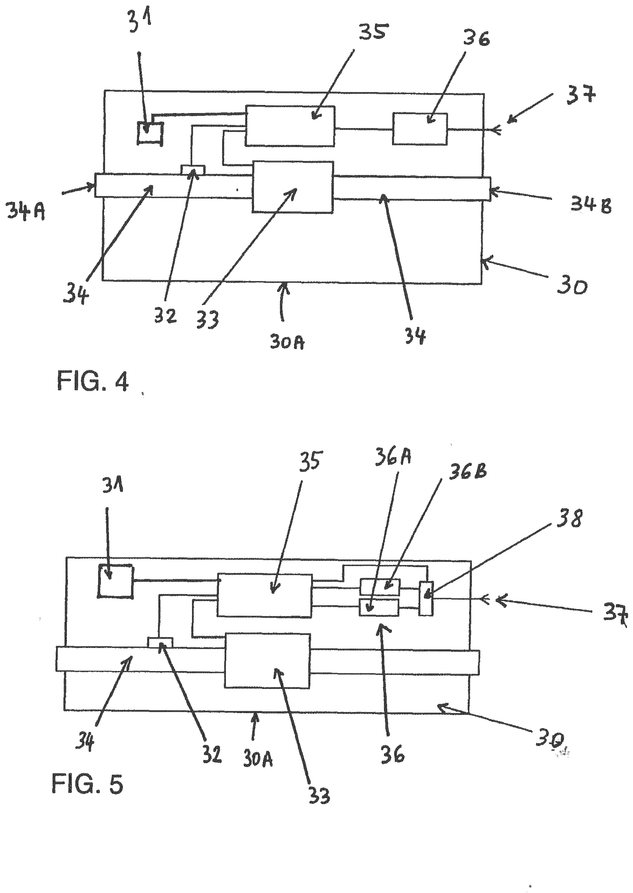

Le canal d'entrée 4 et le conduit d'évacuation de gaz 27 sont en communication avec le conduit de gaz 34 montré sur les Figures 4-8.

On comprend donc qu'à partir d'un champ de vitesse symétrique en 2 dimensions, on crée un tourbillon dont la localisation, i.e. les zones Z1 et Z2, va osciller avec une fréquence proportionnelle à la valeur du débit du fluide qui y circule. En plaçant des microphones ou des organes/capteurs de mesure de pression en dehors du conduit du fluide, c'est-à-dire au dessus des zones Z1, Z2 où se forment les tourbillons, i.e. les « vortex », on peut mesurer la présence ou non d'une dépression du gaz.

Le débitmètre de l'invention permet de déterminer le débit de gaz qui y circule pour des valeurs de débits situées entre 0,5 et 10 L/min.

L'ensemble du système est compris dans un boitier 30A visible en Figure 3 à 8.

Des moyens de pilotage, encore appelés module de pilotage et de traitement de signal 35 (cf. Fig. 4-8), telle une carte électronique à microprocesseur, en particulier à microcontrôleur, sont reliés électriquement aux capteurs de pression ou microphones de manière à recueillir et exploiter les signaux de mesure de pression en extrayant la fréquence d'oscillation pour ensuite en déduire un débit de gaz.

La Figure 2 est une représentation tridimensionnelle du débitmètre de la Figure 1 permettant de visualiser la localisation des orifices de mesure 24, 25 dans le plafond 28 de la chambre de reflux 2.

La Figure 3 schématise une installation d'oxygénothérapie comprenant une source de gaz respiratoire 44, qui est ici une bouteille de gaz, et une interface de distribution de gaz 43 permettant de distribuer le gaz respiratoire à un patient, tel qu'ici des canules nasales, et un appareil de traitement médical 30 selon l'invention, tel un appareil de suivi ou d'observance (Fig. 4-8). L'appareil de traitement médical 30 selon l'invention est agencé sur le trajet de gaz, par exemple sur une canalisation flexible 42, convoyant le gaz de la source de gaz respiratoire 41 aux canules nasales alimentant le patient en gaz respiratoire.

La Figure 4 est un schéma de principe d'un mode de réalisation de l'architecture générale d'un appareil 30 de suivi ou d'observance d'un traitement d'oxygénothérapie selon l'invention. Il comprend un boitier rigide 30A, par exemple en plastique, incorporant un conduit 34 servant à véhiculer un gaz respiratoire, un débitmètre 33 à oscillation fluidique, tel que décrit ci-avant, qui est agencé sur le conduit 34, un premier capteur de pression absolue 31 pour mesurer la pression ambiante, c'est-à-dire la pression atmosphérique, et un second capteur de pression absolue 32 pour mesurer la pression absolue dans le conduit 34, lequel est placé en contact direct avec le conduit 34, avant ou après le débitmètre 33 à oscillation fluidique selon l'invention.

Le flux de gaz circule dans le conduit 34, qui constitue un circuit de circulation de gaz interne traversant le boitier 30A, entre un orifice d'entrée 34A et un orifice de sortie 34B.

Le module de pilotage et de traitement de signal 35, telle une (ou plusieurs) carte électronique, est relié électriquement aux capteurs 31, 32 et au débitmètre 33 de manière à récupérer et traiter les données de mesure opérées par les capteurs 31, 32 et le débitmètre 33.

Une source d'énergie, telle une batterie électrique ou une pile (non montrée), permet d'alimenter le module de pilotage et de traitement 35 en courant électrique.

L'appareil de traitement médical 30 de la Figure 4 est équipé, selon la présente invention, de moyens de télécommunications à longue distance, à savoir un module de télécommunication 36, 37, permettant de transmettre à longue distance des données mesurées par les capteurs 31, 32, qui opèrent des mesures notamment sur le circuit de gaz 34. 'Longue distance' s'entend comme une portée de 100 m à 1 km en milieu urbain dense avec au moins le récepteur ou l'émetteur situé à l'intérieur d'un bâtiment, et/ou une portée de 100 m à 5 km ou plus, voire jusqu'à 30 km, en milieu rural ou en espace dégagé. Alternativement, on peut l'exprimer aussi à travers la notion de sensibilité réalisable pour les récepteurs, par exemple meilleure que -120 dB.

L'électronique embarquée, c'est-à-dire le module de pilotage et de traitement de signal 35, assure le traitement et la mémorisation des signaux de mesure provenant des capteurs 31, 32, et le pilotage du module de télécommunication 36, 37, en particulier d'un modem radio 36 ou *'transceiver'* associé à une antenne 37 émettrice/réceptrice servant à transmettre les signaux traités provenant du module de pilotage et de traitement de signal 35.

Le modem radio 36 assure notamment les fonctions de modulation et démodulation radio, et d'adaptation d'impédance avec l'antenne 37. L'antenne 37 peut être externe au boîtier 30A comme représenté sur la Figure 4 ou, selon un autre mode de réalisation, interne ou intégrée au boitier 30A.

Selon l'invention, le modem radio 36 fonctionne selon une technologie de télécommunication à longue portée, à basse consommation et bas débit par comparaison avec les technologies 2G, 3G et 4G par exemple.

De préférence, le modem radio 36 fonctionne selon une technologie de modulation à étalement de spectre de type LoRa, éventuellement associée au protocole d'accès LoRaWAN, ou une modulation à bande ultra-étroite appelée « UNB » (pour *Ultra-Narrow Band*), en particulier une modulation de type Sigfox associée aux protocoles d'accès sur le réseau du même nom. Dans tous les cas, selon l'invention, le modem radio 36 opère des modulations dans la bande ISM à 868 MHz en Europe, dans la bande ISM à 433 MHz en Europe et Afrique, dans la bande ISM à 2.4 GHz à l'échelle mondiale, dans la bande ISM à 915 MHz aux USA, ou dans la bande ISM à 784 MHz en Chine.

De façon alternative, on peut aussi utiliser des modulations et les protocoles associés sur des réseaux de type EC-GSM, NB-IoT et LTE-M, qui sont généralement mis en oeuvre sur des réseaux d'opérateurs publics et sur des bandes de fréquences réservées aux opérateurs qui sont différentes de celles précédemment citées, en particulier des longueurs d'onde radio associées différentes et des dimensions caractéristiques des antennes.

Selon l'invention, la notion de «bas débit» s'entend par exemple comme un débit de transmission de 100 bits par seconde (cas de Sigfox) à 1 Mégabit par seconde (cas du LTE-M Cat0), ou une partie de cette plage de valeurs.

Les différents composants de l'appareil 30, en particulier le conduit de gaz 34, le débitmètre à oscillation fluidique 33, le premier 31 et le second 32 capteur de pression, le module de pilotage et de traitement de signal 35 et tout ou partie du module de télécommunication 36, 37, sont agencés dans un boitier 30A ou capotage rigide.

De manière particulièrement avantageuse, pour des raisons de redondance et de fiabilisation de la fonction de télécommunication, on propose aussi selon la présente invention, un appareil 30 de traitement médical capable d'utiliser plusieurs technologies de télécommunication alternatives, c'est-à-dire différentes.

Ainsi, la Figure 5 illustre un second mode de réalisation permettant de mettre en oeuvre une telle redondance, c'est-à-dire plusieurs technologies de télécommunication alternatives.

Pour ce faire, le module de télécommunication 36, 37 comprend plusieurs modems radio 36 ou *'transceivers* ', à savoir ici un premier modem radio 36A et un deuxième modem radio 36B agencés en parallèles l'un de l'autre, qui sont associés à une antenne 37 émettrice/réceptrice.

Ces modems radio 36A, 36B permettent d'implémenter deux technologies de télécommunication différentes, par exemple l'un peut fonctionner sur un réseau LoRa et l'autre sur un réseau SigFox.

Le module de pilotage et de traitement de signal 35 embarque ici un microcontrôleur capable d'implémenter les protocoles associés aux deux technologies radio alternatives.

Dit autrement, le module de pilotage et de traitement de signal 35 commande alternativement le premier modem radio 36A dédié à la première technologie radio et le deuxième modem radio 36B dédié à la seconde technologie radio, pour transmettre ou recevoir des données par le biais de l'antenne 37.

En fait, l'antenne 37 est commutée sur l'un ou l'autre des modems radio 36A, 36B par le biais d'un dispositif de commutation, tel un commutateur 38 ou « switch », lequel commutateur 38 est commandé par le module de pilotage et de traitement de signal 35, en particulier par un microcontrôleur intégré dans le module de pilotage et de traitement de signal 35.

Un étage d'adaptation d'impédance (non représenté) peut se trouver en amont ou en aval du commutateur 38 et permet de maximiser la fraction de la puissance du signal au niveau du modem qui est transmise à l'antenne, lors d'une émission, et la fraction de la puissance du signal au niveau l'antenne qui est transmise à au modem, lors d'une réception.

Lorsque les deux technologies radio fonctionnent à des bandes de fréquences différentes, l'antenne 37 est avantageusement une antenne à large bande, par exemple une antenne spirale, log-périodique, fractale, ou autre, capable de recevoir ou transmettre, avec de bonnes propriétés en termes d'efficacité et de diagramme de rayonnement, des signaux sur les différentes bandes de fréquences associées.

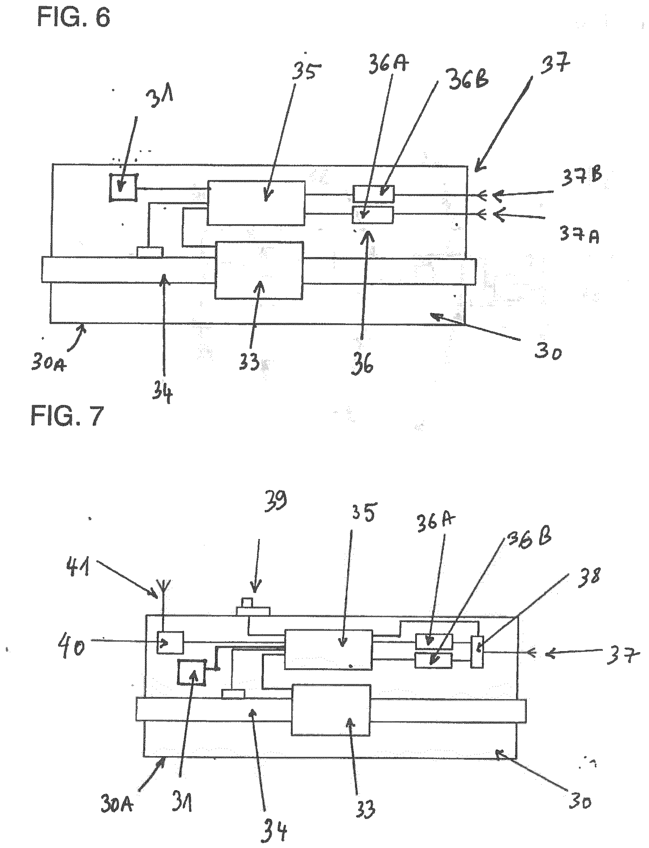

La Figure 6 illustre un troisième mode de réalisation analogue à celui de la Figure 5, à l'exception du fait que le module de télécommunication 36, 37 comprend ici plusieurs antennes 37 émettrices/réceptrices, à savoir ici une première 37A et une seconde 37B antenne.

Comme on le voit, chaque antenne 37A, 37B est reliée électriquement à l'un des modems radio 36A, 36B, à savoir que la première antenne 37A est connectée au premier modem radio 36A dédié à la première technologie radio, alors que la deuxième antenne 37B est connectée au deuxième modem radio 36B dédié à la seconde technologie radio.

Dans ce cas, le commutateur 38 a été supprimé car est inutile. La commutation d'une technologie radio à une autre se fait par pilotage direct par le module de pilotage et de traitement de signal 35, en particulier par le microcontrôleur intégré dans le module de pilotage et de traitement de signal 35.

Par exemple, les protocoles de transmission des données suivants peuvent être mis en oeuvre, seuls ou en combinaison les uns avec les autres :
- le microcontrôleur du module de pilotage et de traitement de signal 35 transmet systématiquement la même série de données d'abord selon une première technologie radio en utilisant le modem radio 36A, puis selon une seconde technologie radio en utilisant le modem radio 36B.
- le microcontrôleur du module de pilotage et de traitement de signal 35 réalise périodiquement une analyse de la qualité de la liaison radio établie avec chaque technologie implémentée, par mesure de la qualité de réception RSSI (pour Received Signal Strength Inticator = Indicateur de Force de Signal Reçu) et/ou SNR (Signal-Noise Ratio = Rapport de Signal de Bruit) d'un message envoyé à l'appareil selon chacune des technologies, et utilise la technologie associée à la meilleure qualité de liaison jusqu'à la prochaine analyse. La notion de meilleure qualité de liaison peut aussi être comprise au sens d'une moyenne pondérée ou non, sur un horizon fini ou non, des qualités de liaisons mesurées par le passé.... Le microcontrôleur réalise alors, selon chaque technologie, l'envoi d'un message comportant une demande d'accusé de réception, pour provoquer l'envoi d'un message vers l'appareil 30.
- le choix de la technologie à utiliser est réalisé une fois pour toute à l'initialisation ou à la réinitialisation de l'appareil 30 sur son lieu d'utilisation, au terme d'une seule phase d'analyse de la meilleure qualité de liaison obtenue
- sur une période de temps donnée, le microcontrôleur transmet P % des données selon une technologie et 100 - P% selon l'autre, la valeur P étant fixée à l'avance et mémorisée. Le microcontrôleur reçoit, selon l'une ou l'autre des liaisons radio, la consigne d'utiliser systématiquement l'une ou l'autre des liaisons, ou d'utiliser une valeur de P modifiée.
- le microcontrôleur réalise 100% de la transmission des données selon l'une des technologies, et périodiquement l'envoi d'un message sans donnée de mesure (et éventuellement porteur de données d'autodiagnostic et d'identification du dispositif) selon l'autre technologie, à savoir d'un message de type signal de présence ou « *heartbeat* » (i.e. battement de coeur) permettant à un dispositif d'indiquer la continuité de son fonctionnement, indépendamment de la transmission de données signifiantes du point de vue applicatif, ou de maintien en fonction ou « *keepalive* » (i.e. garder en vie) permettant de conserver une session active sans échanger de données signifiantes du point de vue applicatif.

La Figure 7 illustre un quatrième mode de réalisation qui est lui aussi analogue à celui de la Figure 5.

Comme précédemment, le module de pilotage et de traitement de signal 35 comprend un microcontrôleur pilotant deux modems radio 36A, 36B spécifiques chacun d'une technologie radio, partageant l'accès à une antenne 37 unique à travers un dispositif de commutation ou commutateur 38 également piloté par le microcontrôleur.

Un commutateur, sélecteur ou interface de saisie additionnel 39, externe ou logé dans le boitier 30A, fournit au module de pilotage et de traitement de signal 35, en particulier au microcontrôleur, une consigne quand à la logique de transmission à utiliser, la technologie à utiliser systématiquement ou la valeur d'un paramètre d'une logique de transmission.

Alternativement ou de manière complémentaire, on prévoit une antenne additionnelle 41 associée à un troisième modem radio 40 relié électriquement au module de pilotage et de traitement de signal 35, de manière à permettre une réception par le module de pilotage et de traitement de signal 35, en particulier le microcontrôleur, d'une consigne quand à la logique de transmission à utiliser, la technologie à utiliser systématiquement, ou la valeur d'un paramètre d'une logique de transmission, au moyen d'une technologie de communication à courte portée telle que : WiFi, Bluetooth, Bluetooth Smart / Bluetooth Low Energy, NFC, Ant / Ant + ou analogue.

Bien entendu, l'antenne 37 pourrait être dissociée en deux antennes 37A, 37B distinctes spécifiques chacune d'une technologie radio, comme dans le mode de réalisation de la Figure 6.

De même, il serait aussi possible de mutualiser les antennes 37, 41, notamment si les technologies radio associées à ces antennes 37, 41 utilisent sensiblement la même bande de fréquence, par exemple LoRa à 2.4 Ghz et Bluetooth à 2.4 GHz, ou si l'antenne 37 est une antenne large-bande, par exemple spirale, fractale, log-périodique ... dont la plage de fréquence utile couvre également celle pour laquelle l'antenne additionnelle 41 est adaptée.

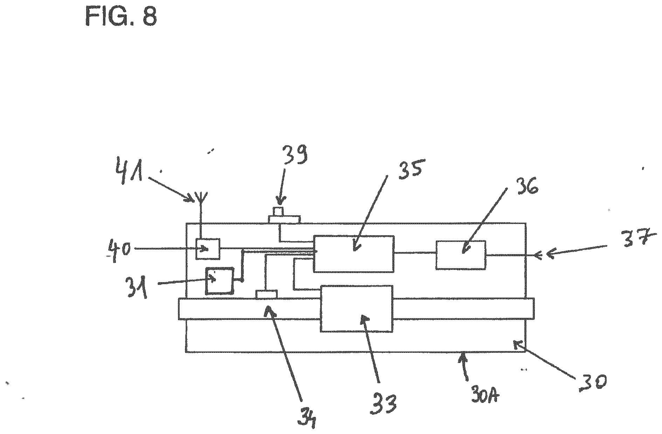

La Figure 8 illustre un cinquième mode de réalisation qui est basé sur une combinaison de tout ou partie des modes de réalisation illustrés sur les Figures 4 et 7 précédentes.

Dans ce cas, l'appareil 30 intègre un unique modem radio 36, comme sur la Figure 4, qui a la capacité logicielle et matérielle d'opérer des télécommunications selon les deux technologies désirées. Par exemple, un composant SX1272 de Semtech éventuellement associé à une référence de fréquence externe (TCXO), peut être piloté pour réaliser des communications selon les technologies LoRa et Sigfox.

Avantageusement, il intègre aussi un commutateur additionnel 39 externe ou logé dans le boitier 30A, et/ou une voie de communication supplémentaire, comme sur la Figure 7, incluant une antenne additionnelle 41 reliée à un modem radio additionnel 40. Toutefois, là encore, il serait aussi possible de mutualiser les antennes 37, 41.

D'une façon générale, quel que soit le mode de réalisation considéré, dans le cas où la technologie de télécommunication utilisée est:
- du type EC-GSM, NB-IoT ou LTE-M, l'appareil 30 de l'invention utilisera préférentiellement une logique d'accumulation des données de mesure au long de plusieurs phases de mesure, grâce à la fonction de mémorisation de l'électronique embarquée, avant de réaliser la transmission de ces données de manière agrégée en un minimum de message (idéalement, 1) et ce, afin d'optimiser la consommation énergétique de la fonction de télécommunication.
- du type Sigfox ou présente des débits sensiblement similaires, par exemple LoRa dans son réglage de débit minimal, on préférera une logique de transmission de la mesure dès qu'une phase de mesure est terminée, ceci afin d'utiliser les temps morts entre les phases de mesure pour respecter les temps morts entre les transmissions imposés sur les bandes ISM.

L'utilisation de commutateurs externes/internes et/ou de voie de communication séparée pour modifier les paramètres utilisés par le microcontrôleur du module de pilotage et de traitement de signal pour gérer les logiques de transmission reste possible dans le cas ou une seule technologie radio longue portée est intégrée. Il en va de même pour ce qui est de la mutualisation des antennes utilisées pour la longue portée et du paramétrage à courte portée.

Bien entendu, bien que les modes de réalisation des Figures 4 à 8 présentent un appareil 30 à un ou deux modems, il va de soi qu'il pourrait en intégrer davantage, par exemple trois ou plus, de manière à pouvoir bénéficier de plus de technologies de communications à longue portée.

L'appareil 30 à débitmètre à oscillation fluidique et système de communication à longue portée de l'invention est particulièrement bien adapté à une utilisation pour le suivi ou l'observance d'un traitement d'oxygénothérapie d'un patient à domicile, l'appareil 30 étant alors relié, d'une part, à une source de gaz respiratoire et, d'autre part, à une interface de distribution de gaz, tel un masque respiratoire, une canule nasale ou analogue, servant à fournir du gaz respiratoire, typiquement de l'oxygène gazeux, au patient.

## Revendications

1. Appareil de traitement médical (30), notamment de suivi ou d'observance d'un traitement d'oxygénothérapie, comprenant :
- un conduit de gaz (34) apte à acheminer un flux gazeux,
- un débitmètre agencé sur le conduit de gaz (34),
- au moins un module de pilotage et de traitement (35) de signal, relié électriquement au débitmètre à oscillation fluidique (33), configuré pour récupérer et traiter les signaux de mesure délivrés par le débitmètre à oscillation fluidique (33), et
- au moins un module de télécommunication (36, 37) relié électriquement au module de pilotage et de traitement (35) de signal et configuré pour transmettre des signaux de mesure provenant du module de pilotage et de traitement (35) de signal à au moins un réseau de communication, ledit au moins un module de télécommunication (36, 37) comprenant au moins un modem radio (36 ; 36A, 36B) associé à au moins une antenne émettrice/réceptrice (37 ; 37A, 37B)
**caractérisé en ce que** :
- le débitmètre est un débitmètre à oscillation fluidique (33), et
- ledit au moins un module de télécommunication (36, 37) est configuré pour présenter une sensibilité inférieure ou égale à -110 dBm et une valeur d'affaiblissement de couplage (MCL) supérieure ou égale à 130 dB avec ledit au moins un réseau de communication considéré.

2. Appareil selon la revendication 1, **caractérisé en ce que** le modem radio (36) est configuré pour opérer au moins une modulation à étalement de spectre, de préférence au moins une modulation de type LoRa, éventuellement associée à un protocole d'accès de type LoRaWAN.

3. Appareil selon la revendication 1, **caractérisé en ce que** le modem radio (36) est configuré pour opérer au moins une modulation à bande ultra-étroite, de préférence au moins une modulation de type Sigfox, éventuellement associée à un protocole d'accès Sigfox.

4. Appareil selon la revendication 1, **caractérisé en ce que** le modem radio (36) est configuré pour opérer au moins une modulation et un protocole ou une pile de protocoles associés sur au moins un réseau choisi parmi les réseaux EC-GSM, NB-IoT et LTE-M

5. Appareil selon l'une des revendications précédentes, **caractérisé en ce que** le modem radio (36) est configuré pour opérer selon un débit de transmission compris entre 100 bits par seconde et 1 Mégabit par seconde.

6. Appareil selon l'une des revendications précédentes, **caractérisé en ce que** la sensibilité est inférieure ou égale à -120 dBm et/ou la valeur de MCL est supérieure ou égale à 135 dB, de préférence la sensibilité est inférieure ou égale à -125 dBm et/ou la valeur de MCL est supérieure ou égale à 140 dB.

7. Appareil selon l'une des revendications précédentes, **caractérisé en ce que** le module de télécommunication (36, 37) comprend plusieurs modems radio (36 ; 36A, 36B) agencés en parallèles l'un de l'autre, et associés à au moins une antenne (37) émettrice/réceptrice.

8. Appareil selon la revendication 7, **caractérisé en ce que** le module de pilotage et de traitement de signal (35) commande alternativement le premier modem radio (36A) et le deuxième modem radio (36B), de préférence via un dispositif de commutation (38).

9. Appareil selon la revendication 7, **caractérisé en ce que** le module de télécommunication (36, 37) comprend plusieurs antennes (37 ; 37A, 37B) émettrices/réceptrices, chaque antenne (37 ; 37A, 37B) étant reliée électriquement à l'un des modems radio (36 ; 36A, 36B).

10. Appareil selon la revendication 7, **caractérisé en ce qu'**il comprend un commutateur additionnel (39) configuré pour fournir au module de pilotage et de traitement de signal (35), une consigne quant à la logique de transmission à utiliser, la technologie à utiliser systématiquement ou la valeur d'un paramètre d'une logique de transmission de manière à permettre une sélection d'un des modems radio (36 ; 36A, 36B).

11. Appareil selon l'une des revendications précédentes, **caractérisé en ce que** ledit au moins modem radio est configuré pour opérer des modulations dans la bande ISM à 868 MHz, à 915 MHz, à 433 MHz, à 784 MHz ou à 2.4 GHz.

12. Appareil selon la revendication 8, **caractérisé en ce qu'**il comprend en outre une antenne additionnelle (41) associée à un troisième modem radio (40) relié électriquement au module de pilotage et de traitement de signal (35).

13. Installation d'oxygénothérapie comprenant une source de gaz respiratoire (41), une interface de distribution de gaz (43) permettant de distribuer le gaz respiratoire à un patient, et un appareil de traitement médical (30) selon l'une des revendications précédentes.

## Patentansprüche

1. Medizinische Behandlungseinrichtung (30), insbesondere zur Überwachung oder zur Beobachtung einer Sauerstofftherapiebehandlung, umfassend:
- eine Gasleitung (34), die geeignet ist, eine Gasströmung zu befördern,
- einen Durchflussmesser, der auf der Gasleitung (34) angeordnet ist,
- mindestens ein Signalansteuerungs- und -verarbeitungsmodul (35), das an den Durchflussmesser nach dem Schwingstrahlprinzip (33) elektrisch angeschlossen ist, konfiguriert, um die vom Durchflussmesser nach dem Schwingstrahlprinzip (33) ausgegebenen Messsignale zu sammeln und zu verarbeiten, und
- mindestens ein Telekommunikationsmodul (36, 37), das an das Signalansteuerungs- und -verarbeitungsmodul (35) elektrisch angeschlossen ist und konfiguriert ist, um vom Signalansteuerungs- und -verarbeitungsmodul (35) stammende Messsignale an mindestens ein Kommunikationsnetzwerk zu übertragen, wobei das mindestens eine Telekommunikationsmodul (36, 37) mindestens ein Funkmodem (36; 36A, 36B) umfasst, das mindestens einer Sende-/Empfangsantenne (37; 37A, 37B) zugeordnet ist,
**dadurch gekennzeichnet, dass**:
- der Durchflussmesser ein Durchflussmesser nach dem Schwingstrahlprinzip (33) ist, und
- das mindestens eine Telekommunikationsmodul (36, 37) konfiguriert ist, um eine Empfindlichkeit kleiner als oder gleich -110 dBm und einen Koppeldämpfungswert (MCL) größer als oder gleich 130 dB mit dem mindestens einen betrachteten Kommunikationsnetzwerk aufzuweisen.

2. Einrichtung nach Anspruch 1, **dadurch gekennzeichnet, dass** das Funkmodem (36) konfiguriert ist, um mindestens eine Spektrumsspreizungsmodulation, vorzugsweise mindestens eine Modulation vom Type LoRa, eventuell einem Zugangsprotokoll vom Typ LoRaWAN zugeordnet, vorzunehmen.

3. Einrichtung nach Anspruch 1, **dadurch gekennzeichnet, dass** das Funkmodem (36) konfiguriert ist, um mindestens eine Ultraschmalbandmodulation, vorzugsweise mindestens eine Modulation vom Typ Sigfox, eventuell einem Sigfox-Zugangsprotokoll zugeordnet, vorzunehmen.

4. Einrichtung nach Anspruch 1, **dadurch gekennzeichnet, dass** das Funkmodem (36) konfiguriert ist, um mindestens eine Modulation und ein Protokoll oder einen Stapel von zugeordneten Protokollen auf mindestens einem Netzwerk vorzunehmen, das unter den Netzwerken EC-GSM, NB-loT und LTE-M ausgewählt ist.

5. Einrichtung nach einem der vorstehenden Ansprüche, **dadurch gekennzeichnet, dass** das Funkmodem (36) konfiguriert ist, um gemäß einem Übertragungsdurchfluss, der zwischen 100 Bits pro Sekunde und 1 Megabit pro Sekunde enthalten ist, vorzunehmen.

6. Einrichtung nach einem der vorstehenden Ansprüche, **dadurch gekennzeichnet, dass** die Empfindlichkeit kleiner als oder gleich -120 dBm ist und/oder der MCL-Wert größer als oder gleich 135 dB ist, die Empfindlichkeit vorzugsweise kleiner als oder gleich -125 dBm und/oder ist der MCL-Wert größer als oder gleich 140 dB ist.

7. Einrichtung nach einem der vorstehenden Ansprüche, **dadurch gekennzeichnet, dass** das Telekommunikationsmodul (36, 37) mehrere Funkmodems (36; 36A, 36B) umfasst, die parallel zueinander angeordnet sind und mindestens einer Sende--/Empfangsantenne (37) zugeordnet sind.

8. Einrichtung nach Anspruch 7, **dadurch gekennzeichnet, dass** das Signalansteuerungs- und -verarbeitungsmodul (35) alternierend das erste Funkmodem (36A) und das zweite Funkmodem (36B), vorzugsweise über eine Schaltervorrichtung (38), steuert.

9. Einrichtung nach Anspruch 7, **dadurch gekennzeichnet, dass** das Telekommunikationsmodul (36, 37) mehrere Sende-/Empfangsantennen (37; 37A, 37B) umfasst, wobei jede Antenne (37; 37A, 37B) elektrisch an eines der Funkmodems (36; 36A, 36B) angeschlossen ist.

10. Einrichtung nach Anspruch 7, **dadurch gekennzeichnet, dass** sie einen zusätzlichen Schalter (39) umfasst, der konfiguriert ist, um dem Signalansteuerungs-und -verarbeitungsmodul (35) eine Anweisung in Bezug auf die zu verwendende Übertragungslogik, die systematisch zu verwendende Technologie oder den Wert eines Parameters einer Übertragungslogik zu liefern, um eine Auswahl eines der Funkmodems (36; 36A, 36B) zu ermöglichen.

11. Einrichtung nach einem der vorstehenden Ansprüche, **dadurch gekennzeichnet, dass** das mindestens Funkmodem konfiguriert ist, um Modulationen im ISM-Band bei 868 MHz, bei 915 MHz, bei 433 MHz, bei 784 MHz oder bei 2,4 GHz vorzunehmen.

12. Einrichtung nach Anspruch 8, **dadurch gekennzeichnet, dass** sie weiter eine zusätzliche Antenne (41) umfasst, die einem dritten Funkmodem (40) zugeordnet ist, das elektrisch an das Signalansteuerungs- und -verarbeitungsmodul (35) angeschlossen ist.

13. Sauerstofftherapieanalage, umfassend eine Atemgasquelle (41), eine Gasverteilungsschnittstelle (43), die es ermöglicht, das Atemgas an einen Patienten zu verteilen, und eine medizinische Behandlungseinrichtung (30) nach einem der vorstehenden Ansprüche.

## Claims

1. Medical treatment apparatus (30), in particular for monitoring or observing an oxygen therapy treatment, comprising:
- a gas line (34) suitable for transporting a gas flow,
- a flowmeter arranged on the gas line (34),
- at least one signal control and processing module (35), electrically connected to the fluidic oscillation flowmeter (33), configured to recover and process measurement signals delivered by the fluidic oscillation flowmeter (33), and
- at least one telecommunication module (36, 37) electrically connected to the signal control and processing module (35) and configured to transmit measurement signals, coming from the signal control and processing module (35), to at least one communication network, said at least one telecommunication module (36, 37) comprising at least one radio modem (36; 36A, 36B) associated with at least one transmitting/receiving antenna (37; 37A, 37B)
**characterised in that**:
- the flowmeter is a fluidic oscillation flowmeter (33), and
- said at least one telecommunication module (36, 37) is configured to have a sensitivity less than or equal to -110 dBm and a maximum coupling lost (MCL) value greater than or equal to 130 dB with said at least one communication network in question.

2. Apparatus according to claim 1, **characterised in that** the radio modem (36) is configured to operate at least one spread-spectrum modulation, preferably at least one LoRa modulation, optionally associated with an LoRaWAN access protocol.

3. Apparatus according to claim 1, **characterised in that** the radio modem (36) is configured to operate at least one ultra-narrowband modulation, preferably at least one Sigfox modulation, optionally associated with a Sigfox access protocol.

4. Apparatus according to claim 1, **characterised in that** the radio modem (36) is configured to operate at least one modulation and one protocol or a stack of associated protocols on at least one network chosen from among the EC-GSM, NB-loT and LTE-M networks.

5. Apparatus according to one of the preceding claims, **characterised in that** the radio modem (36) is configured to operate at a transmission rate between 100 bits per second and 1 Megabit per second.

6. Apparatus according to one of the preceding claims, **characterised in that** the sensitivity is less than or equal to -120 dBm and/or the MCL value is greater than or equal to 135 dB, preferably the sensitivity is less than or equal to -125 dBm and/or the MCL value is greater than or equal to 140 dB.

7. Apparatus according to one of the preceding claims, **characterised in that** the telecommunication module (36, 37) comprises a plurality of radio modems (36; 36A, 36B) arranged in parallel with one another and associated with at least one transmitting/receiving antenna (37).

8. Apparatus according to claim 7, **characterised in that** the signal control and processing module (35) alternately controls the first radio modem (36A) and the second radio modem (36B), preferably via a switching device (38).

9. Apparatus according to claim 7, **characterised in that** the telecommunication module (36, 37) comprises a plurality of transmitting/receiving antennas (37; 37A, 37B), each antenna (37; 37A, 37B) being electrically connected to one of the radio modems (36; 36A, 36B).

10. Apparatus according to claim 7, **characterised in that** it comprises an additional switch (39) configured to supply, to the signal control and processing module (35), an instruction regarding the transmission logic to be used, the technology to be systematically used or the value of a transmission logic parameter so as to enable a selection of one of the radio modems (36; 36A, 36B).

11. Apparatus according to one of the preceding claims, **characterised in that** said at least one radio modem is configured to operate modulations in the ISM band at 868 MHz, at 915 MHz, at 433 MHz, at 784 MHz or at 2.4 GHz.

12. Apparatus according to claim 8, **characterised in that** it further comprises an additional antenna (41) associated with a third radio modem (40) electrically connected to the signal control and processing module (35).

13. Oxygen therapy facility comprising a respiratory gas source (41), a gas dispensing interface (43) enabling the respiratory gas to be dispensed to a patient, and a medical treatment apparatus (30) according to one of the preceding claims.
